(19) Europäisches Patentamt
European Patent Office
Office européen des brevets

(11) **EP 2 241 249 A1**

(12) **EUROPÄISCHE PATENTANMELDUNG**

(43) Veröffentlichungstag:
20.10.2010 Patentblatt 2010/42

(51) Int Cl.:
*A61B 5/00* (2006.01)    *A61B 5/021* (2006.01)

(21) Anmeldenummer: 10156626.3

(22) Anmeldetag: 16.03.2010

(84) Benannte Vertragsstaaten:
AT BE BG CH CY CZ DE DK EE ES FI FR GB GR HR HU IE IS IT LI LT LU LV MC MK MT NL NO PL PT RO SE SI SK SM TR
Benannte Erstreckungsstaaten:
AL BA ME RS

(30) Priorität: 15.04.2009 DE 102009002399

(71) Anmelder: Biotronik CRM Patent AG
6341 Baar (CH)

(72) Erfinder:
• **Krämer, Thomas**
**90425, Nürnberg (DE)**

• **Lippert, Michael**
**91522, Ansbach (DE)**
• **Skerl, Olaf**
**18209, Bad Doberan (DE)**
• **Czygan, Gerald**
**91054, Buckenhof (DE)**
• **Paule, Stefan**
**96117, Drosendorf (DE)**

(74) Vertreter: **Lindner-Vogt, Karin L.**
**Biotronik SE & Co. KG**
**Corporate Intellectual Properties**
**Woermannkehre 1**
**12359 Berlin (DE)**

(54) **Vorrichtung und Verfahren zum Verarbeiten physiologischer Messwerte**

(57) Die Erfindung betrifft eine Monitoring-Vorrichtung mit einem Signaleingang für Signale, die Messwerte eines oder mehrerer physiologischer Parameter repräsentieren und einer mit dem Signaleingang verbundenen Bewertungs- und Verarbeitungseinheit. Die Bewertungs- und Verarbeitungseinheit ist ausgebildet, unter den eingehenden Werten einzelne Werte für die weitere Verarbeitung nach einem oder mehreren der folgenden Kriterien derart auszuwählen oder unterschiedlich zu gewichten, dass Messwerte, deren Validität angezweifelt werden muss, nicht ausgewählt oder mit sehr niedriger Gewichtung versehen werden.

FIG. 4

EP 2 241 249 A1

**Beschreibung**

Die Erfindung betrifft eine Vorrichtung und ein Verfahren zum Auswerten von Sensormesswerten, die zur Prädiktion einer Dekompensation dienen sollen. Als Sensormesswerte werden hier die Ausgangssignale von Sensoren für physiologische Parameter bezeichnet.

Bei Patienten, die unter Herzinsuffizienz leiden, kann es in unregelmäßigen Abständen zu einer akuten Verschlechterung ihres Zustandes kommen, verbunden mit einem völligen Zusammenbruch der Leistungsfähigkeit, Atemnot und Erstickungsangst. In diesem Fall muss eine sofortige Hospitalisierung mit Notfallbehandlung erfolgen.

Diese Ereignisse werden durch eine so genannte "Dekompensation" hervorgerufen, bei der das Herz nicht mehr in der Lage ist, eine ausreichende Pumpleistung zur Verfügung zu stellen. Dieses Ereignis kündigt sich einige Wochen vorher durch einen (sich selbst verstärkenden) Anstieg des Druckes im Lungenkreislauf an, und damit verbunden einer zunehmenden Einlagerung von Wasser in das Lungengewebe, was jedoch vom Patienten in der Regel nicht rechtzeitig wahrgenommen wird.

Das Zustandekommen einer Dekompensation, verbunden mit einer großen Belastung des Patienten und des Herz-Kreislaufsystems selbst, verschlechtert das Krankheitsbild der Herzinsuffizienz beträchtlich. Die Lebenserwartung des Patienten kann durch rechtzeitiges Erkennen von sich anbahnenden Dekompensationen wesentlich erhöht werden. Durch die mögliche Vermeidung der durch einen kritischen Zustand des Patienten erforderlichen Hospitalisierung können die Kosten der medizinischen Versorgung reduziert werden. Notwendig hierfür ist ein Gerät, das bei Anbahnung einer Dekompensation rechtzeitig vor der akuten Phase eine Warnung erzeugt. Daraufhin können frühzeitig entsprechende Therapiemaßnahmen eingeleitet und eine Hospitalisierung verhindert werden.

Patienten, die unter Herzversagen leiden, werden zunehmend mit einem implantierten Defibrillator (ICD) versorgt, da Teile dieser Patientengruppe auch ein erhöhtes Risiko für lebensbedrohliche Tachyarrhythmien tragen.

Die verwendeten ICD sind dabei als Einkammer- oder Zweikammersysteme ausgeführt. Etwa 2/3 aller Patienten mit Herzversagen leiden unter systolischem Herzversagen, bei dem die Effizienz des Blutauswurfs stark verschlechtert ist (z. B. hervorgerufen durch einen Linksschenkel-Block). Solche Patienten werden heute zunehmend mit einem Implantat therapiert, das durch synchrone Stimulation des rechten und linken Herzens die Synchronität der Kontraktion des linken und rechten Herzens wiederherstellt. Sie erhalten eine so genannte kardiale Resynchronisations-Therapie (CRT), bei der ein Schrittmacher oder ICD separate Elektroden zum rechten und zum linken Ventrikel (über den Coronar-Sinus) erhält.

Ein physikalischer Parameter, mit dem die zunehmende Ansammlung von Flüssigkeit im Thorax ermittelt werden kann, ist die transthorakale elektrische Impedanz. Nimmt der Flüssigkeitsgehalt im Lungengewebe zu, sinkt die gemessene Impedanz. Mit Hilfe eines Implantats und der implantierten Elektroden kann diese transthorakale Impedanz auf einfache Weise gemessen werden. Dadurch kann eine Dekompensation frühzeitig vor der akuten Phase erkannt und damit Patient oder Arzt gewarnt werden, um daraufhin therapeutische Maßnahmen einzuleiten.

Bekannt sind Vorrichtungen, die die transthorakale Impedanz zwischen dem Implantatsgehäuse und/oder einer oder mehreren kardialen Elektroden messen, um eine Flüssigkeitsansammlung in der Lunge zu detektieren (US 5,957,861, US 6,076,015, US 6,454,719, US 2006/0041280, US 2006/0258952, US 2006/0264776). Allgemein werden die Impedanzwerte über einen längeren Zeitraum (z. B. über 24 Stunden) gemittelt, um die durch Herz- und Atemzyklus und zirkadiane Schwankungen verursachten Impedanzvariationen auszugleichen. Diese gemittelten Werte dienen als Basis für die Früherkennung von sich anbahnenden Lungenödemen; siehe Yu CM, Wang L, Chau E, Chan RH, Kong SL, Tang MO, Christensen J, Stadler RW, Lau CP. "Intrathoracic impedance monitoring in patients with heart failure: correlation with fluid status and feasibility of early warning preceding hospitalization." Circulation 2005;112(6):841-8.

Sekundäre Effekte, die nicht durch die Entstehung eines Lungenödems bedingt sind, können die transthorakale Impedanz ebenfalls stark beeinflussen und eine Flüssigkeitsansammlung vortäuschen oder sie verdecken. Diese Störungen müssen in ihrem Einfluss auf die Detektion eines entstehenden Lungenödems kompensiert werden.

Eine dieser Störungen kann durch Schwankungen der Blutleitfähigkeit, verursacht beispielsweise durch einen sich verändernden Hämatokrit oder den variierenden Elektrolytgehalt im Blut, entstehen.

Um den Einfluss der Blutleitfähigkeit zu verringern, werden Systeme beschrieben, die die Blutleitfähigkeit bestimmen und zur Korrektur der transthorakalen Impedanz verwenden (US 2006/0041280, US 2006/0258952, US 2006/0264776).

Die Impedanzen von Lungengewebe und von Blut weisen eine unterschiedliche Frequenzcharakteristik auf. Es wurde ein System beschrieben, das aus diesem Grunde die Impedanzmessung bei verschiedenen Frequenzen durchführt, um den Einfluss der Blutleitfähigkeit zu minimieren (EP 1 665 983).

Auch Änderungen der Körperlage (z. B. Aufstehen oder Hinlegen) führen zu einer Flüssigkeitsumverteilung im Körper und damit zu einer temporären Veränderung des Flüssigkeitsgehalts in der Lunge. Diese Änderungen im Flüssigkeitsgehalt werden von der transthorakalen Impedanz prinzipbedingt wiedergegeben und möglicherweise als entstehendes Lungenödem bewertet. Aus diesem Grunde wurden Systeme beschrieben, die die Körperlage oder deren Änderung erfassen und in die Bewertung der transthorakalen Impedanz einbeziehen (US 2006/0041280, US 2006/0258952, US 2006/0264776). Weitere Störungen können durch Flüssigkeitsansammlungen in der Implantattasche oder Migration des Implantats entstehen.

Alle diese Lösungen haben den Nachteil, dass zusätzliche Sensoren oder zusätzliche Messungen notwendig sind. Die dafür notwendige Energie kann besonders bei Langzeitimplantaten zu einer merklichen Verringerung der Lebensdauer führen.

Beschrieben wurden auch Systeme, die die Atmungstätigkeit selbst überwachen, um aus der ermittelten Atemfrequenz oder dem Atemrhythmus (z. B. Kurzatmigkeit, Cheyne-Stokes-Atmung, Schlafapnoe) auf eine Verschlechterung des Krankheitszustandes zu schließen (US 5,876,353, US 5,957,861, US 6,076,015, US 6,449,509, US 6,454,719, US 2006/0258952).

Die Atemtätigkeit wird allerdings erst in einem relativ späten Stadium des Lungenödems merklich beeinflusst; siehe Zipes, D.P. et al. [ed.]: Braunwald's Heart Disease; Elsevier, 2005. Darüber hinaus wird die Atmung aber auch von vielen anderen Faktoren beeinflusst, wie z. B. körperliche Belastung, Sprechen und allgemeiner Gesundheitszustand (NYHA). Dadurch ist die eindeutige Früherkennung eines Lungenödems rein aus der Atmung sehr fehleranfällig. So wird beispielsweise die Abhängigkeit der Impedanz von der Atmung auch ausgenutzt, um das Atemminutenvolumen zu bestimmen, was zur Abschätzung der metabolischen Belastung dient und zur Ansteuerung eines frequenzadaptiven Schrittmachers Verwendung findet (US 6,076,015, US 6,449,509).

Weiterhin wurden Verfahren beschrieben, die über eine intrakardial gemessene Impedanz hämodynamische Größen bestimmen und aus deren Veränderung eine Veränderung des Krankheitszustandes ableiten. Dabei wird die Dynamik des Herzschlages selbst bestimmt und Schwankungen, die durch die Atmung und durch andere Einflüsse hervorgerufen werden, beispielsweise durch Mittelung beseitigt (Zima, E., et al. "Determination of left ventricular volume changes by intracardiac conductance using a biventricular electrode configuration." Europace 8.7 (2006): 537-44; Stahl, C., et al. "Intracardiac Impedance Monitors Hemodynamic Deterioration in a Chronic Heart Failure Pig Model." J.Cardiovasc.Electrophysiol. 18 (2007): 985-90; EP 1 510 173).

Bekannt sind auch Systeme, die mehrere verschiedene Parameter kombinieren, um den Krankheitsverlauf besser beurteilen und die Unsicherheiten eines einzelnen Parameters kompensieren zu können (US 5,876,353, US 5,957,861, US 2006/0258952, US 2006/0264776).

Die bekannten Lösungen haben verschiedene Nachteile.

Ein entscheidender Nachteil der bekannten Lösungen liegt darin begründet, dass die transthorakale elektrische Impedanz nicht nur durch die zunehmende Flüssigkeitsansammlung in der Lunge, sondern auch von vielen anderen Faktoren beeinflusst wird. Durch die Einflüsse dieser sekundären Faktoren reduzieren sich die Sensitivität und die Spezifität bei der Lungenwasserdetektion. Deshalb müssen die sekundären Einflussfaktoren durch zusätzliche Parameter oder zusätzliche Messgrößen teilweise auch anderer Sensoren (z. B. Körperlage, intrakardiale Drucksensoren) kompensiert werden. Zusätzliche Messungen erhöhen den Aufwand und den Energieverbrauch. Durch den erhöhten Energieverbrauch verringert sich, vor allem bei Langzeitimplantaten, die Lebensdauer des Systems. Auch die bekannten Atmungsparameter sind von vielen anderen Faktoren abhängig und allein genommen wenig spezifisch. So ist auch hierbei die Verwendung zusätzlicher Parameter notwendig.

Der Erfindung liegt die Aufgabe zugrunde, eine Vorrichtung und ein Verfahren anzugeben, die die Nachteile des Standes der Technik weitestgehend vermeiden, leicht zu realisieren sind und insbesondere eine möglichst hohe Sensitivität und Spezifität bieten.

Erfindungsgemäß wird diese Aufgabe durch eine Monitoring-Vorrichtung mit einem Signaleingang für Signale, die Messwerte eines oder mehrerer physiologischer Parameter repräsentieren und einer mit dem Signaleingang verbundenen Bewertungs- und Verarbeitungseinheit gelöst. Die Bewertungs- und Verarbeitungseinheit ist ausgebildet, unter den eingehenden Werten einzelne Werte für die weitere Verarbeitung nach einem oder mehreren der folgenden Kriterien derart auszuwählen oder unterschiedlich zu gewichten, dass

Messwerte, deren Validität angezweifelt werden muss, nicht ausgewählt oder mit sehr niedriger Gewichtung versehen werden, wenn:

- die Variabilität (z. B. Standardabweichung oder relative Standardabweichung) eines Messwert der selbst ein Mittelwert aus vielen Einzelwerten ist, über einem vorgegebenen entsprechendem Grenzwert liegt,
- ein die Aktivität eines Patienten kennzeichnendes Sensorsignal wie ClosedLoopStimulations-Ratensteuersignal, Akzelerometerausgangswert, Atemfrequenz, Atemtiefe, und/oder Minutenvolumen über einem vorgegebenen entsprechendem Grenzwert liegt,
- ein Signal oder mehrere Signale einen instabilen Zustand eines Patienten wie eine ventrikuläre Tachykardie anzeigt, oder
- eine antitachykarde Therapie ausgeführt wird, wie beispielsweise eine antitachykarde Stimulation oder die Abgabe eines Defibrillationsschocks.

Wenn aufgrund der obigen Kriterien die Gewichtung einer Messung unterhalb einer vorgegebenen oder adaptiven Schwelle liegen wird, kann deren Auswertung abgebrochen werden, um Energie, Verarbeitungszeit, Bandbreite bei der Datenübertragung, und TrendSpeicher zu sparen. Falls vor der Durchführung einer Sensormessung bereits aus anderen Parametern, oder während der Messung bereits aus Teilmessungen (z. B. die erste Minute einer 10 Minuten andauernden Messung), nach obigen Kriterien eine Gewichtung unterhalb einer bestimmten Schwelle zu erwarten ist, kann diese Messung aus denselben Gründen auch ganz entfallen, bzw. abgebrochen werden. Abgebrochene oder entfallende Messungen tragen damit auch zu einer Ersparnis in Bezug auf Energie und/oder Verarbeitungszeit und/oder Datenübertragung und/oder Trendspeicher bei.

Weiterhin kann eine Messung, wenn aufgrund der obigen Kriterien die Gewichtung einer Messung unterhalb einer vorgegebenen oder adaptiven Schwelle liegen würde, die Messung zeitlich, um bis zu einer vorher festgelegten maximalen Verspätung, verschoben werden, bis die Kriterien wieder im sinnvollen Bereich liegen. Auf diese Weise kann, wahlweise zu einer Energieersparnis, die Vollständigkeit einer Messreihe mit validen Messpunkten verbessert werden. Welche der Methoden gewählt wird, Weglassen oder Verschieben, hängt von den Prioritäten von Energieersparnis und Vollständigkeit der Messreihen ab.

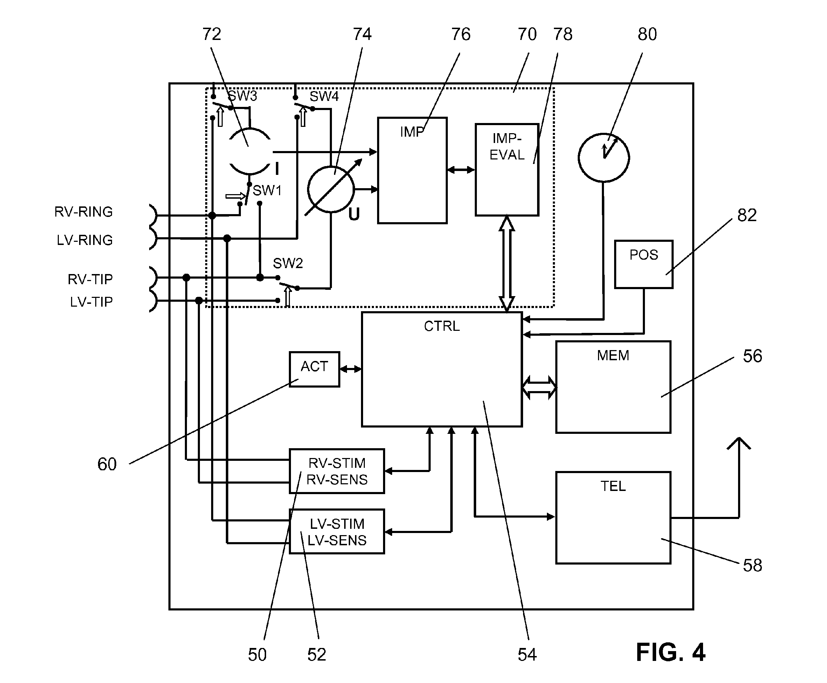

Eine Erläuterung des ClosedLoopStimulations-Ratensteuersignal findet sich in der Beschreibung zu Figur 4.

Vorzugsweise ist die Bewertungs- und Verarbeitungseinheit ausgebildet, unter den eingehenden Werten einzelne Werte für die weitere Verarbeitung nach einem oder mehreren der folgenden Kriterien derart auszuwählen oder unterschiedlich zu gewichten, dass Messwerte unter Bedingungen, die eine hohe Konstanz der Bedingungen und damit eine gute Vergleichbarkeit aufweisen, mit einer hohen Wichtung versehen und bevorzugt ausgewertet werden.

Zum Beispiel ist die Bewertungs- und Verarbeitungseinheit ausgebildet, Messwerte, die in einem vorgegebenen Tageszeitbereich, z. B. Schlafenszeit, aufgenommen werden, mit einer hohen Wichtung zu versehen und bevorzugt auszuwerten.

Zusätzlich oder alternativ kann die Bewertungs- und Verarbeitungseinheit ausgebildet sein, Messwerte, die einem vorgegebenen Herzfrequenzbereich (z. B. BasicRate(BR) ... BR + 10bpm) zugeordnet sind, mit einer hohen Wichtung zu versehen und bevorzugt auszuwerten.

Die Bewertungs- und Verarbeitungseinheit kann auch ausgebildet sein, Messwerte, die einem vorgegebenen Belastungsbereich (z. B. CLS-Stimulationsratensteuersignal-Bereich) zugeordnet sind, mit einer hohen Wichtung zu versehen und bevorzugt auszuwerten.

Gemäß einer weiteren bevorzugten Ausführungsvariante ist die Bewertungs- und Verarbeitungseinheit ausgebildet, Messwerte, die mit einem Anteil atrialen Flimmerns innerhalb einer bestimmten Zeitspanne (atrial burden) einhergehen, der unterhalb oder oberhalb eines vorgegebenen Grenzwertes liegt, mit einer hohen Wichtung zu versehen und bevorzugt auszuwerten.

Die Bewertungs- und Verarbeitungseinheit kann vorzugsweise ausgebildet sein, Messwerte, die mit einer Atmungsamplitude und Atemfrequenz in einem vorbestimmten oder adaptiv ermittelten Intervall einhergehen, mit einer hohen Wichtung zu versehen und bevorzugt auszuwerten.

Gemäß einer besonders bevorzugten Ausführungsvariante ist die Bewertungs- und Verarbeitungseinheit ausgebildet, unter den eingehenden Werten einzelne Werte für die weitere Verarbeitung nach einem oder mehreren der folgenden Kriterien derart auszuwählen oder unterschiedlich zu gewichten, dass Messwerte, die unter Bedingungen gewonnen wurden, bei denen das Messsignal eine hohe Empfindlichkeit gegenüber dem zu beobachtenden Effekt zeigt, bevorzugt ausgewertet und mit einer hohen Wichtung versehen werden.

In diesem Zusammenhang kann die Bewertungs- und Verarbeitungseinheit ausgebildet sein, Messwerte, die mit einem Anteil atrialen Flimmerns innerhalb einer bestimmten Zeitspanne (atrial burden) einhergehen, der oberhalb eines vorgegebenen Grenzwertes liegt, mit einer hohen Wichtung zu versehen und bevorzugt auszuwerten.

Vorzugsweise besitzt die Monitoring-Vorrichtung einen Eingang für ein Positursensorsignal, das eine Änderung der Positur (z. B. liegende oder aufrechte Positur) anzeigt. Die Bewertungs- und Verarbeitungseinheit ist dann vorzugsweise ausgebildet, Messwerte, die nach Posituränderung in eine liegende Positur aufgenommen werden, mit einer hohen Wichtung zu versehen und bevorzugt auszuwerten.

Die Bewertungs- und Verarbeitungseinheit kann auch ausgebildet sein, Messwerte, die unter hoher körperlicher oder physischer Belastung aufgenommen werden, mit einer hohen Wichtung zu versehen und bevorzugt auszuwerten.

Zusätzlich oder alternativ kann die Bewertungs- und Verarbeitungseinheit auch ausgebildet sein, Messwerte, die bei hoher Herzfrequenz aufgenommen werden, mit einer hohen Wichtung zu versehen und bevorzugt auszuwerten.

Bei einer Monitoring-Vorrichtung mit einem Eingang für ein Positursensorsignal, das eine liegende oder aufrechte Positur anzeigt, und einem Eingang für ein Tageszeitsignal, ist die Bewertungs- und Verarbeitungseinheit vorzugsweise ausgebildet, Messwerte, die morgens nach Wechsel von einer liegenden Positur zu einer aufrechten Positur (also beim morgendlichen Aufstehen) aufgenommen werden, mit einer hohen Wichtung zu versehen und bevorzugt auszuwerten.

Die Bewertungs- und Verarbeitungseinheit ist vorzugsweise mit einer mit einer Impedanzerfassungseinheit zum Bestimmen eines Impedanzsignals, das einen intrakardialen Impedanzverlauf repräsentiert und einer Impedanzauswertungseinheit verbunden, die aus dem zeitlichen Verlauf des Impedanzsignals ein Aktivitätssignal bestimmt, welches hohe körperliche oder physische Belastung anzeigt (CLS-Signal).

Erfindungsgemäß wird die eingangs genannte Aufgabe auch durch ein Verfahren zum Verarbeiten von Messwerten physiologischer Parameter gelöst, bei dem unter den eingehenden Werten einzelne Werte für die weitere Verarbeitung nach einem oder mehreren der folgenden Kriterien derart ausgewählt oder unterschiedlich gewichtet werden, dass Messwerte, deren Validität angezweifelt werden muss, nicht ausgewählt oder mit sehr niedriger Gewichtung versehen werden, wenn:

- die Variabilität (z. B. Standardabweichung oder relative Standardabweichung) eines Messwert, der selbst ein Mittelwert aus vielen Einzelwerten sein kann, über einem vorgegebenen entsprechendem Grenzwert liegt,
- ein die Aktivität eines Patienten kennzeichnendes Sensorsignal wie ClosedLoopStimulations-Rate, Akzelerometerausgangswert, Atemfrequenz, Atemtiefe, und/oder Minutenvolumen über einem vorgegebenen entsprechendem Grenzwert liegt,
- ein Signal oder mehrere Signale einen instabilen Zustand eines Patienten wie eine ventrikuläre Tachykardie anzeigt, oder
- eine antitachykarde Therapie ausgeführt wird (ATP, Schock).

Die Erfindung soll nun anhand eines Ausführungsbeispiels mit Bezug auf die Figuren näher erläutert werden. Von diesen zeigt:

Fig. 1    ein erfindungsgemäßes Implantat in der Außendarstellung

Fig. 2:    ein Blockschaltbild eines ersten Ausführungsbeispiels des erfindungsgemäßen Implantats;

Fig. 3    ein Blockschaltbild einer beispielhaften Ausgestaltung eines Impedanzauswerte- einheit des erfindungsgemäßen Implantats;

Fig. 4    ein Blockschaltbild eines zweiten Ausführungsbeispiels des erfindungsgemäßen Implantats;

Fig. 5    eine beispielhafte Darstellung eines zeitlichen Impedanzverlaufs mit zugehöri- gem Mittelwert und dem Atmungssignal;

Fig. 6    Verringerung des Effektivwertes des Atmungssignals während der Herausbil- dung eines Lungenödems;

Fig. 7    den Verlauf des Trends der mittleren Impedanz;

Fig. 8    ein erfindungsgemäßes Implantat in Verbindung mit einem Patientengerät und einem Service-Center;

Fig. 9:    ein Beispiel für die Auswahl von Messungen während atrialer Fibrillation (AF); und

Fig. 10:    ein Beispiel für eine hohe Gewichtung von Tagesmittelwerten mit hohem AF- Anteil.

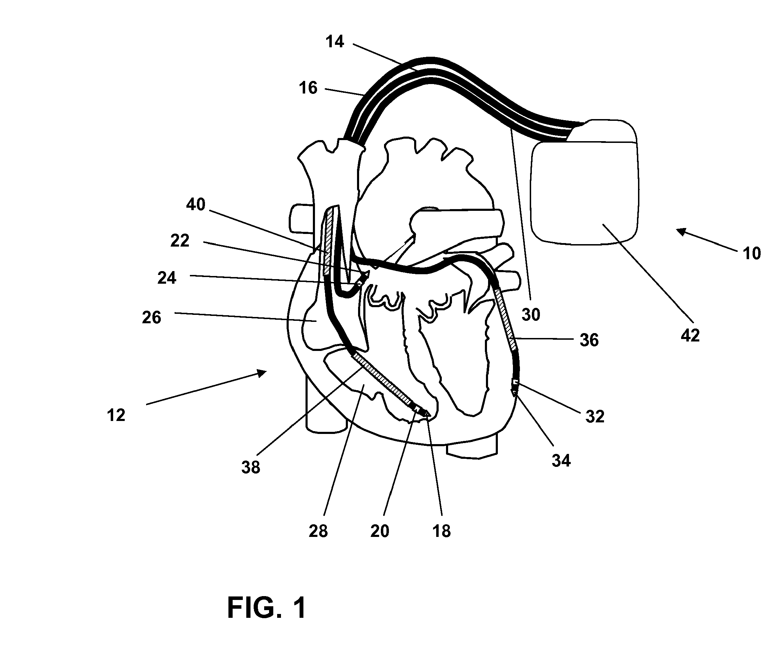

Fig. 1 zeigt ein Implantat 10 in Form eines biventrikulären Dreikammer-Herzschrittmachers und/oder Cardioverters/Defibrillators (ICD). An diesen sind drei Elektrodenleitungen angeschlossen, nämlich eine rechtsatriale Elektrodenleitung 14, eine rechtsventrikuläre Elektrodenleitung 16 und eine linksventrikuläre Elektrodenleitung 30. Im implantierten Zustand endet die rechtsatriale Elektrodenleitung 14 im rechten Atrium 26 eines Herzens 12. Die rechtsventrikuläre Elektrodenleitung 16 endet im rechten Ventrikel 28 des Herzens 12 und die linksventrikuläre Elektrodenleitung 30 reicht über den Coronarsinus des Herzens 12 bis zum linken Ventrikel des Herzens.

Die rechtsatriale Elektrodenleitung 14 trägt an ihrem distalen Ende eine rechtsatriale Spitzenelektrode 22 und in geringem Abstand davon eine rechtsatriale Ringelektrode 24. In ähnlicher Weise trägt die rechtsventrikuläre Elektrodenleitung 16 an ihrem distalen Ende eine rechtsventrikuläre Spitzenelektrode 18 und wenig entfernt davon eine rechtsventrikuläre Ringelektrode 20. Auch am distalen Ende der linksventrikulären Elektrodenleitung 30 ist eine linksventrikuläre Spitzenelektrode 34 und wenig entfernt davon eine linksventrikuläre Ringelektrode 32 angebracht. Diese Elektroden dienen der Aufnahme elektrischer Potenziale in der jeweiligen Herzkammer sowie der Abgabe von Stimulationsimpulsen an die jeweilige Herzkammer im normalen Schrittmacherbetrieb. Diese braucht an dieser Stelle nicht mehr erläutert zu werden.

Die rechtsventrikuläre Elektrodenleitung 16 trägt außerdem noch eine im implantierten Zustand im rechten Ventrikel angeordnete rechtsventrikuläre Schockwendel 38 sowie eine im implantierten Zustand in der Vena cava superior befindliche zweite Schockwendel 40. Auch an der linksventrikulären Elektrodenleitung 30 ist eine linksventrikuläre Schockwendel 36 angebracht. Die Schockwendeln dienen im Bedarfsfall als Defibrillations-Elektroden zur Abgabe von Defibrillationsschocks. Auch dies braucht an dieser Stelle nicht näher erläutert zu werden.

Für die vorliegende Erfindung ist hingegen von Bedeutung, dass das Implantat 10 ausgebildet ist, über sein metallisch leitendes Gehäuse 42 und beispielsweise die rechtsventrikuläre Schockwendel 38 einen schwachen unterschwelligen, also nicht zu Gewebekontraktionen führenden Wechselstrom abzugeben und über die rechtsventrikuläre Spitzenelektrode 18 und einem von dem elektrisch leitenden Gehäuse 42 gebildeten Gegenpol die infolge des eingespeisten Wechselstroms abfallende Spannung zu messen. Dies erlaubt es in an sich bekannter Weise, eine transthorakale Impedanz zu bestimmen.

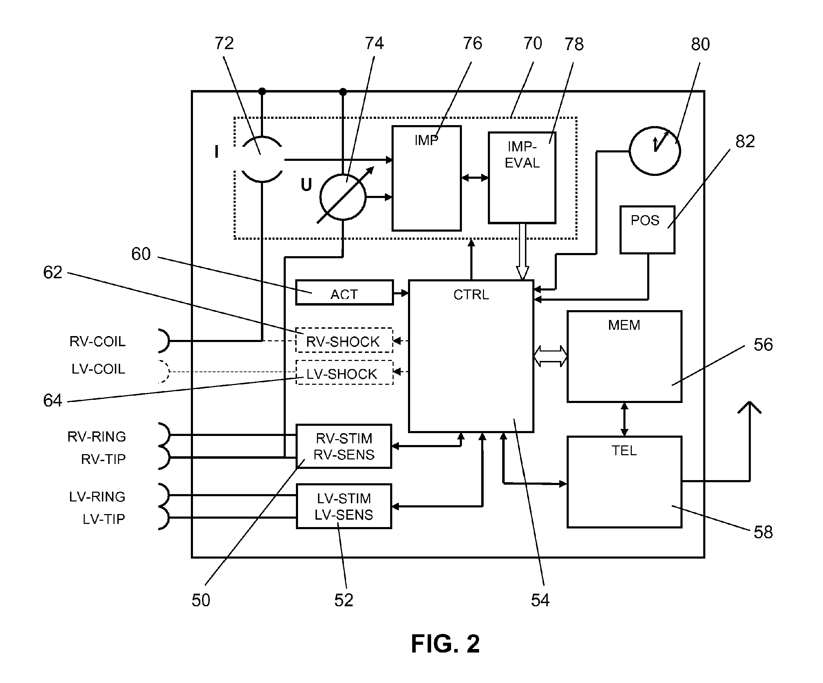

Fig. 2 zeigt in einem schematischen Blockschaltbild das Implantat 10 mit den üblichen Bestandteilen eines Herzschrittmachers/Cardioverters/Defibrillators, nämlich Anschlüssen für die in Fig. 1 dargestellten Elektrodenleitungen sowie mit diesen verbunden, in dem Hohlgehäuse 42 untergebrachten elektrischen Komponenten, wie einem rechtsventrikulären Defibrillationsschockgenerator 62, einem linksventrikulären Defibrillationsschockgenerator 64, einer rechtsventrikuläre Sensing- und Stimulationseinheit 50 (die der Einfachheit halber in dieser Darstellung zu einer Einheit zusammengefasst sind) sowie einer entsprechenden linksventrikulären Stimulations- und Sensingeinheit 52. Diese Komponenten sind mit einer zentralen Steuereinheit 54 verbunden, die außerdem auch mit einem Aktivitätssensor 60 verbunden ist.

Weiterhin ist ein Tageszeitgeber 80 vorgesehen, der ein die jeweilige Tageszeit anzeigendes Tageszeitsignal liefert, so z. B. Tag- und Nachtzeiten unterschieden werden können. Ein Positursensor 82 ist ausgebildet, ein Positursignal aus der Lage des Implantats abzuleiten, um auf diese Weise eine sitzende oder stehende, also aufrechte Positur eines Patienten von einer liegenden Positur unterscheiden zu können.

Ein Speicher 56 dient dem Speicher mit Programmdaten zum Steuern der Steuereinheit 54 sowie zum Speichern von durch das Implantat 10 gewonnenen Messwerten und Betriebsparametern. Die Steuereinheit 54 und der Speicher 56 sind außerdem mit einer Telemetrieeinheit 58 verbunden, die es erlaubt, drahtlos Daten aus dem Implantat 10 an ein externes Patientengerät zu übertragen oder umgekehrt von diesem Daten, beispielsweise neue Steuerparameter, zu empfangen.

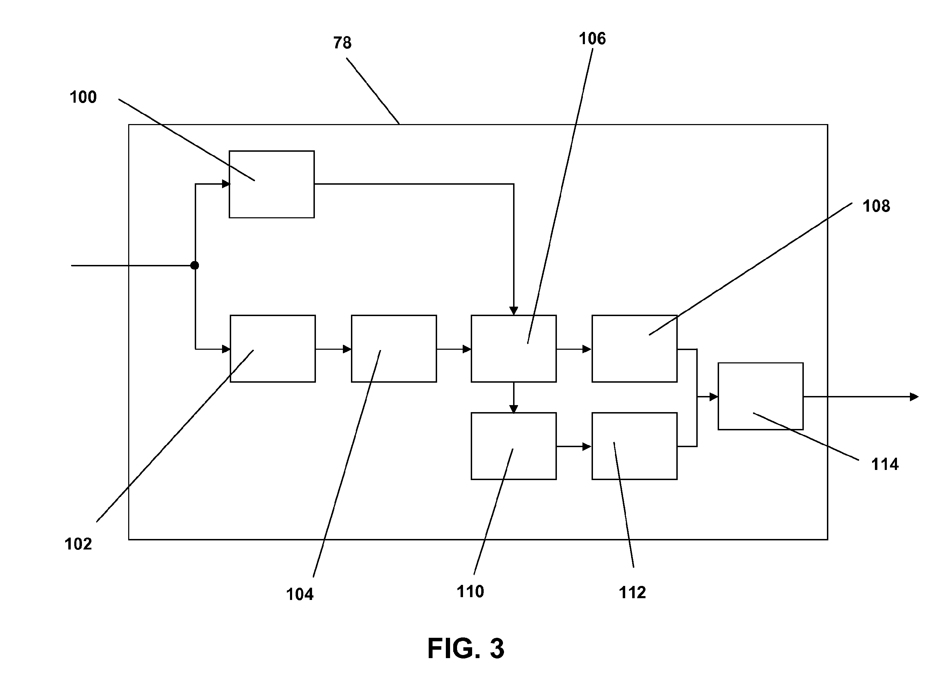

Eine Impedanzerfassungs- und Auswerteeinheit 70 umfasst eine Wechselstromquelle 72 und eine Spannungsmesseinheit 74. Die Wechselstromquelle 72 ist ausgebildet, in an sich bekannter Weise biphasische Stromimpulse zu generieren und über den Anschluss RV-Coil und die rechtsventrikulärer Schockwendel 38 sowie über das Gehäuse 42 an im implantierten Zustand dazwischen liegendes Körpergewebe abzugeben. Die Spannungsmesseinheit 74 misst die aus den abgegebenen Wechselstromimpulsen resultierende Spannung. Die Stärke der abgegebenen Stromimpulse und die daraus resultierende Spannung wird durch eine Impedanzerfassungseinheit 76 in regelmäßigen Zeitabständen ausgewertet, so dass sich eine Zeitfolge von die jeweilige Impedanz repräsentierenden Werten, im Folgenden Impedanzwerte genannt, ergibt, die den zeitlichen Verlauf der Impedanz zwischen Gehäuse 42 des Implantats und dem rechten Ventrikel widerspiegeln. Diese Impedanz ist die transthorakale Impedanz. Zur Auswertung dieses Impedanzverlaufs ist eine Auswerteeinheit 78 vorgesehen, die die Impedanz wie folgt auswertet (siehe Figur 3).

Die von der Impedanzerfassungseinheit 76 stammende Folge von Impedanzwerten wird zunächst einer Digitalfilterung unterzogen, um durch digitale Filterung mit verschiedenen Zeitkonstanten den Mittelwert M des Impedanzverlaufs und ein Atmungssignal R zu bestimmen. Ein erstes Digitalfilter 100 dient dabei der Bestimmung des Mittelwertes M und ein zweites Digitalfilter 102 dient der Bestimmung des Atmungssignals R. Dem Digitalfilter 102 nachgeschaltet ist eine Effektivwert-Bestimmungseinheit 104 zur Bestimmung des Effektivwerts des Atmungssignals R. Dem nachgeschaltet ist wiederum eine Verhältnisbestimmungseinheit 106, die mit einem ersten Eingang mit der Effektivwertbestimmungseinheit 104 und mit einem zweiten Eingang mit dem ersten Digitalfilter 100 verbunden ist und dazu ausgebildet ist, das Verhältnis vom Effektivwert des Atmungssignals R zur mittleren Impedanz M zu bilden. Dieses Verhältnis stellt einen von der Impedanzauswertungseinheit 78 bestimmten Modulationsparameter dar, dessen Parameterwert in einem nachgeschalteten ersten Komparator 108 mit einem Schwellwert als Vergleichswert verglichen wird.

Die Verhältnisbestimmungseinheit 106 ist ausgangsseitig außerdem mit einer Trendbestimmungseinheit 110 verbunden, die jeweils die Differenz zwischen zwei aufeinander folgenden Modulationsparametern bildet und diese Differenz mit einer vorangegangenen Differenz vergleicht. Dieser Vergleich kann ebenfalls durch Differenzbildung erfolgen. Das so gewonnene Ausgangssignal wird einem zweiten Komparator 112 zugeführt, mit dem dieses Ausgangssignal mit einem zweiten Schwellwert als Vergleichswert verglichen wird.

Die Ausgangswerte des ersten Komparators 108 und des zweiten Komparators 112 werden einer Bewertungseinheit 114 zugeführt, die in Abhängigkeit der beiden Vergleichsergebnisse ein Dekompensationswarnsignal generiert oder nicht.

Fig. 4 zeigt eine alternative Ausführungsvariante des Implantats 10, bei der der an das Gewebe abzugebende Wechselstrom einerseits über das Gehäuse 42 des Implantats und andererseits sowohl über die rechtsventrikuläre Ringelektrode 20 als auch die linksventrikuläre Ringelektrode 32 abgegeben wird. Die Spannungsmessung erfolgt einerseits über das Gehäuse 42 des Implantats 10 und andererseits über die rechtsventrikuläre Spitzenelektrode 18 und die linksventrikuläre Spitzenelektrode 34. Weitere Elektrodenkonfigurationen für die Messung der transthorakalen Impedanz sind grundsätzlich bekannt und möglich.

Die Impedanzerfassungs- und Auswerteeinheit 70 umfasst außerdem Schalter SW1, SW2, SW3 und SW4, mit denen die Spannungsmesseinheit 74 wahlweise mit der rechtsventrikulären Spitzenelektrode 18 und dem Gehäuse 42 zum Bestimmen eines transthorakalen Impedanzverlauf verbunden werden kann, oder mit der linksventrikulären Spitzenelektrode 34 und der linksventrikulären Ringelektrode 32, um einen intrakardialen Impedanzverlauf aufzunehmen. Im erstgenannten Fall erfolgt die Stromeinspeisung über die rechtsventrikuläre Ringelektrode 20 und das Gehäuse 42, im letztgenannten Fall über die rechtsventrikuläre Spitzenelektrode 18 und die rechtsventrikuläre Ringelektrode 20. Für die Erfassung der ventrikulären Kontraktionsdynamik zum Bestimmen eins Stimulationsratensteuersignals im Rahmen der ClosedLoop-Stimulation (CLS) erfolgt die Stromeinspeisung und die Spannungsmessung jeweils über die rechtsventrikuläre Spitzenelektrode 18 und das Gehäuse 42 des Implantats 10. Hierfür wird eine Differenz-Fläche zwischen einer aktuellen Impedanzkurve und einer Referenz-Ruhekurve bestimmt und ausgewertet.

Dieses Signal geht auch in die Wichtung der erfassten Messwerte ein.

Die Detektion einer Dekompensation erfolgt beispielsweise durch Auswerten des Zeitverlaufs der intrathorakalen Impedanz Z(t).

Die nachfolgenden Erläuterungen dienen dem tieferen Verständnis der Erfindung und beschreiben weitere mögliche Ausgestaltungen der Impedanzauswerteeinheit 78, die hier bildlich nicht näher dargestellt sind.

Die transthorakale Impedanz schwankt zeitlich um einen Mittelwert M, d. h. die Impedanz wird durch die Herzbewegungen und die Atmung zyklisch moduliert. Der Zeitverlauf der Impedanz Z(t) ist geprägt durch die relativ hochfrequente Herzbewegung, der der langsamere Atemzyklus R überlagert ist.

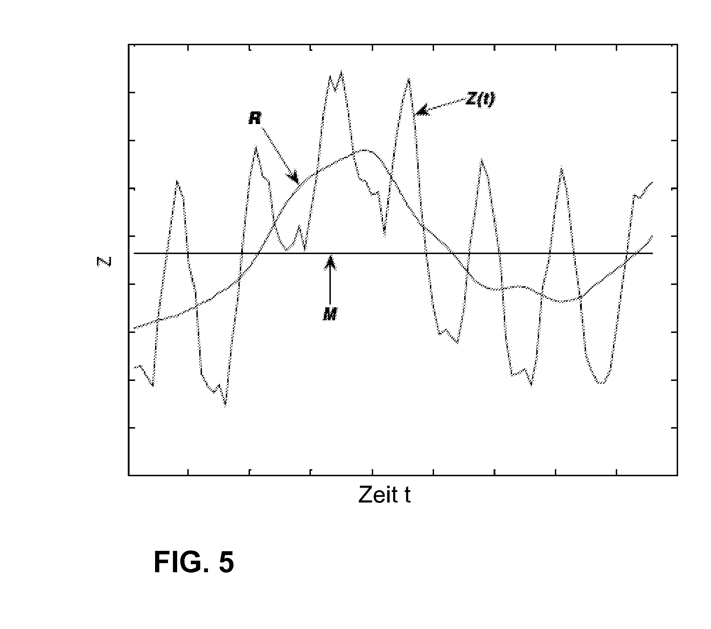

Figur 5 zeigt beispielhaft einen Zeitverlauf der intrathorakalen Impedanz Z(t) mit dem zugehörigen Mittelwert (M) und dem Atmungssignal (R).

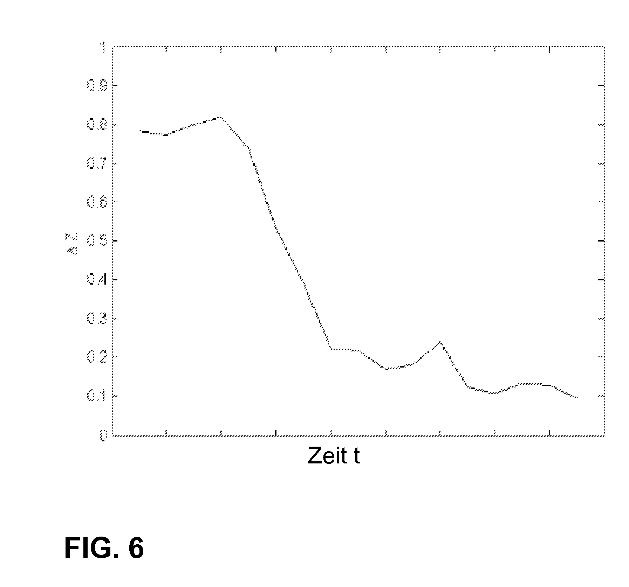

Mit zunehmender Flüssigkeitsansammlung in der Lunge verringert sich bekanntermaßen der mittlere Impedanzwert, was von den bekannten Verfahren zur Lungenödemdetektion genutzt wird. Es wurde aber festgestellt, dass sich dabei ebenfalls die durch die Atmung hervorgerufene Modulation der transthorakalen Impedanz (Atmungssignal; R) deutlich verringert, selbst wenn Atemtiefe und Atemfrequenz unverändert bleiben; siehe Figur 6.

Die in der Lunge angestaute Flüssigkeit schließt das Lungengewebe zunehmend elektrisch kurz. Dadurch verringert sich der Einfluss des wechselnden Luftgehalts in der Lunge auf die Impedanz während eines Atemzyklus. Die Impedanzmodulation ist direkt an die Atmung gekoppelt. Andere Faktoren, wie beispielsweise Änderungen in der Blutleitfähigkeit, können dadurch in ihren Einflüssen getrennt werden, anders als bei der mittleren Impedanz. Veränderungen an der Atmungsmodulation der transthorakalen Impedanz durch den Flüssigkeitsstau in der Lunge sind bereits detektierbar weit bevor die Atmung symptomatisch beeinträchtigt wird.

Um aussagekräftige Parameter der Atmungsmodulation bestimmen zu können, muss die durch die Atmung verursachte Komponente (R) vom Gleichanteil der transthorakalen Impedanz (M) und von der durch den Herzzyklus hervorgerufenen Komponente (Herzsignal) getrennt werden. Dies geschieht durch die Impedanzauswertungseinheit. Diese kann hierfür allgemein bekannte Verfahren verwenden, wie beispielsweise verschiedene Filterverfahren oder die herzzyklussynchrone Messung der Impedanz. Diese Verfahren lösen das Atmungssignal R aus dem Zeitverlauf der transthorakalen Impedanz Z(t) heraus. Aus dem herausgelösten Atmungssignal werden geeignete Parameter abgeleitet, die den Grad der Modulation beschreiben und zur Abschätzung der Flüssigkeitsansammlung in der Lunge dienen können. Vorzugsweise ist die Impedanzauswertungseinheit ausgebildet einen oder mehreren der nachfolgenden Modulationsparameter zu bestimmen:

- Effektivwert oder mittlere Amplitude des Atmungssignals,
- Verhältnis von Effektivwert oder mittlerer Amplitude zur mittleren Impedanz M,
- Relation des Effektivwertes zur Atemfrequenz,
- Interquartilsabstand,

wobei diese Aufzählung nur beispielhaften Charakter hat und weder vollständig noch einschränkend ist. Der Wert eines oder mehrerer dieser Modulationsparameter wird dann von der Impedanzauswertungseinheit mit einem Kriterium, beispielsweise einem Schwellwert, verglichen und über die Detektion einer Dekompensation entschieden (z. B. bei Überschreitung des Schwellwerts). Das Kriterium kann dabei eine fest vorgegebene Größe haben oder patientenindividuell einstellbar sein.

Darüber hinaus kann die Impedanzauswertungseinheit auch ausgebildet sein, die Trends eines oder mehrerer Modulationsparameter über eine geeignete Zeitdauer aufzuzeichnen und auszuwerten. Aus diesen Trends kann die bevorzugte Impedanzauswertungseinheit wiederum geeignete Trendparameter bestimmen, insbesondere einen oder mehrer der nachfolgend aufgeführten Trendparameter:

- die Differenz zum vorhergehenden Parameterwert oder die Änderung dieser Differenz gegenüber der vorhergehenden,

- zirkadiane Schwankungen des oder der Parameter,
- Änderungen der zirkadianen Schwankung des Parameters,
- Änderung der Relation zwischen Atmungsamplitude und Atemfrequenz.

Auch diese Trendparameter werden durch die Impedanzauswertungseinheit wieder mit einem Kriterium verglichen und damit eine Dekompensation detektiert. Weiterhin kann die Impedanzauswertungseinheit ausgebildet sein den Parametertrend mit dem Schwellwert zu verknüpfen. So kann die Impedanzauswertungseinheit beispielsweise aus der Dauer und dem Grad der Unterschreitung des Schwellwertes eine Aussage über eine entstandene Dekompensation ableiten.

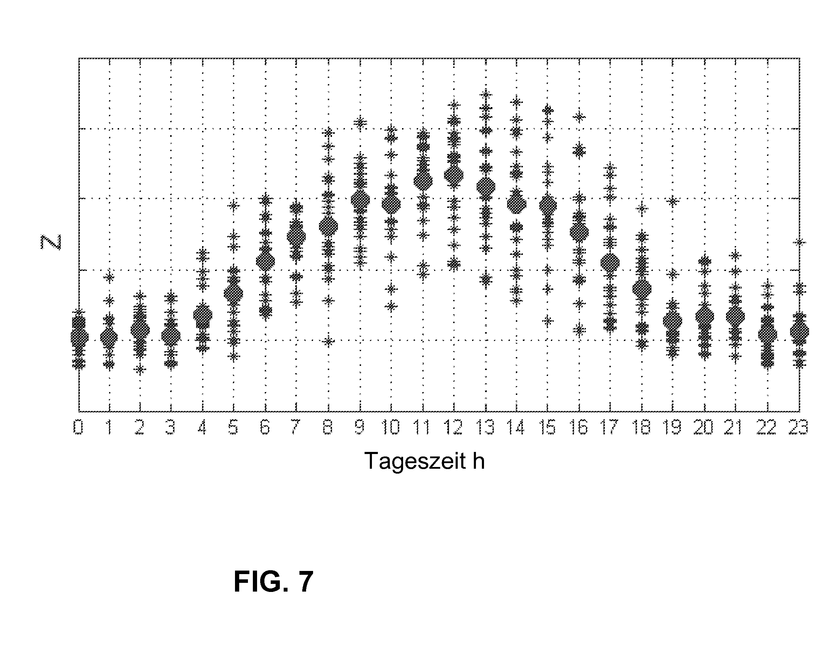

Betrachtet man den Trend der mittleren Impedanz M findet man ebenfalls zeitliche Schwankungen, die auf einen deutlichen zirkadianen (tageszeitabhängigen) Rhythmus der Impedanz hinweisen; siehe Figur 7.

Es ist zu vermuten, dass das zirkadiane Muster der Impedanz auf Flüssigkeitsverlagerungen im Körper zurückzuführen ist, die mit anderen tageszeitabhängigen rhythmologischen (z. B. Herzrate) und/oder hämodynamischen Größen (z. B. Blutdruck) verknüpft sind. Aber auch weitere, z. B. hormonale, Einflussfaktoren können dabei eine Rolle spielen. Daher wird das zirkadiane Muster unter Umständen wesentlich vom Krankheitszustand des Patienten bestimmt, da z. B. Erholungsphasen in der Nacht beim kranken Patienten weniger ausgeprägt sind als im gesunden bzw. weniger kranken Patienten. Daher wird das zirkadiane Signal der Impedanz bzw. daraus abgeleitete Parameter ebenso zur Detektion eines Lungenödems bzw. zur Bewertung der bestehenden bzw. fortschreitenden Herzinsuffizienz herangezogen.

Die Impedanzauswertungseinheit ist vorzugsweise ausgebildet, durch geeignete Methoden, z. B. durch stündliche Mittelung der zyklisch gemessenen Impedanzwerte, das zirkadiane Signal aus der mittleren Impedanz M zu ermitteln.

Beispielsweise werden vom Implantat in regelmäßigen Zeitabständen (z. B. 1 Stunde) Messungen der intrakardialen Impedanz vorgenommen, die jeweils aus vielen Einzelmessung ($Z_i$) bestehen, z. B. 15 Minuten lang pro Herzzyklus eine Impedanzmessung, oder wenige Minuten lang kontinuierlich mit konstanter Sampling-Frequenz, z. B. 32 Hz. Aus jeder der stündlichen Messungen werden ein Mittelwert ($Zmean_h$) und eine Varianz ($Zvar_h$) berechnet. Aus diesen (z. B. n = 24) Messwerten werden jeden Tag eine Reihe von aus der Impedanz abgeleiteten Parametern P berechnet. Pro Parameter ergibt das einen Wert P pro Tag.

Aus diesem zirkadianen Signal bestimmt die Impedanzauswertungseinheit in ihrer bevorzugten Ausführungsform wiederum Parameter (zirkadiane Parameter), die dieses Signal charakterisieren. Vorzugsweise ist die Impedanzauswertungseinheit ausgebildet, einen oder mehrere der nachfolgend genannten zirkadianen Parameter zu bestimmen:

- maximale Tagesschwankung der Impedanz als Differenz zwischen maximalem und minimalem Wert,
- das Verhältnis der Dauer der beiden Halbperioden (oberhalb und unterhalb des Tagesmittelwertes) zueinander,
- Phasenlage der zirkadianen Schwankung, z. B. Uhrzeit des Maximums und/oder Minimums
- Differenz der Impedanzwerte von zwei konstanten Zeitpunkten oder auch die Tag-Nacht-Differenz.

Gemäß einer weiter bevorzugten Ausführungsvariante ist die Impedanzauswertungseinheit ausgebildet, diese zirkadianen Parameter ebenfalls wieder mit einem Kriterium zu vergleichen. Daraus kann eine Aussage über eine entstandene oder drohende Dekompensation abgeleitet bzw. die Entwicklung der zugrunde liegenden Herzinsuffizienz bewertet werden. Auch hier kann die Impedanzauswertungseinheit weiter ausgebildet sein, den Trend eines oder mehrerer zirkadianer Parameter über eine geeignete Zeitdauer zu ermitteln und zirkadiane Trendparameter abzuleiten, die ebenfalls mit einem Kriterium verglichen werden können. Auch kann die Impedanzauswertungseinheit ausgebildet sein, den Trend mit dem Kriterium zu verknüpfen, um eine Aussage über den Grad der Dekompensation zu treffen.

Darüber hinaus kann die Impedanzauswertungseinheit auch ausgebildet sein, einen zirkadianen Parameter oder dessen Trend mit dem Trend eines Modulationsparameters des Atmungssignals oder dem Modulationsparameter selbst zu verknüpfen, beispielsweise

- Phasenverschiebung zwischen zirkadianem Signal und zirkadianem Trend eines Atmungssignalparameters,
- Vergleich der Atmungssignalparameter nur in einem bestimmten Teil des zirkadianen Rhythmus (z. B. immer nur zu bestimmten Uhrzeiten oder zu Zeiten eines Minimums im zirkadianen Signal).

Ebenfalls kann die Impedanzauswertungseinheit ausgebildet sein, die Korrelation zwischen einem oder mehreren Parametern des zirkadianen Signals und Parametern des Atmungssignals zu bewerten und daraus den Grad der Dekompensation abzuleiten.

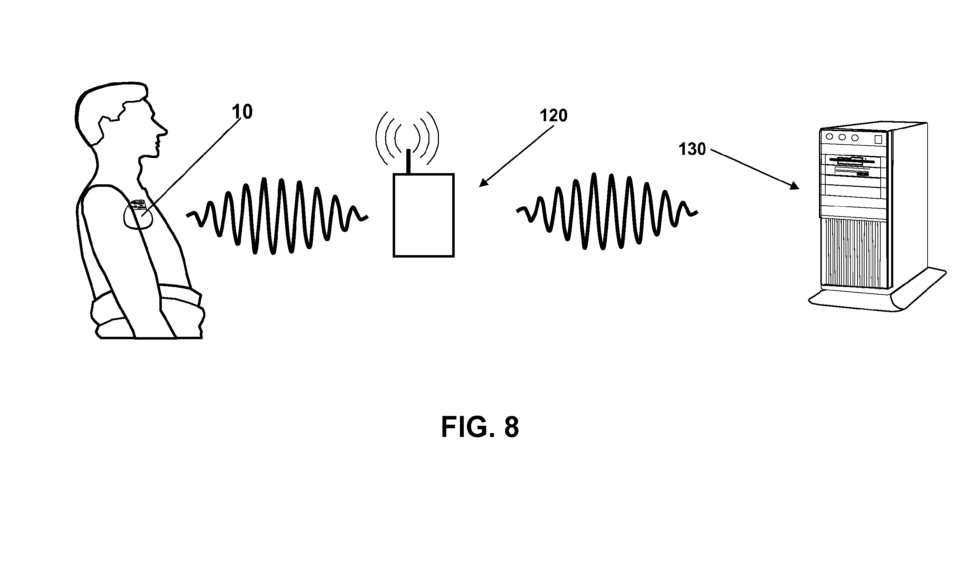

Die Bestimmung der Parameter und Trends und das Prüfen gegen das entsprechende Kriterium können einerseits vollständig im Implantat erfolgen, wenn die Impedanzauswertungseinheit vollständig in das Implantat integriert ist. Wenn die Impedanzauswertungseinheit durch die Prüfung gegen das Kriterium eine Dekompensation detektiert, kann das Implantat einen Alarm generieren (z. B. akustisch oder durch Vibrieren oder eine Alarmmeldung über eine Telemetrieverbindung an ein externes Patientengerät 120 senden; siehe Figur 8) oder selbständig Maßnahmen zur Therapieanpassung durchführen, wie beispielsweise die Freigabe von Medikamenten oder die Anpassung ihrer Dosierung oder die Anpassung der Stimulationsparameter bei Schrittmachern oder ICD.

Andererseits kann das Implantat nur die entsprechenden Parameter bestimmen und diese über eine Telemetrieverbindung an ein externes Patientengerät senden. Im Patientengerät kann dann die eventuelle Ermittlung der Trends und die Prüfung gegen das Kriterium erfolgen. In diesem Fall ist die Impedanzauswertungseinheit wenigstens teilweise Teil des Patientengerätes. Bei Detektion einer Dekompensation kann das Patientengerät einen Alarm generieren (wiederum z. B. akustisch oder auch über eine Datenverbindung den Arzt oder ein Service-Center benachrichtigen) oder selbständig therapeutische Maßnahmen einleiten. Das Patientengerät kann auch die Parameter in geeigneten Zeitintervallen über eine Datenverbindung an ein Service-Center weiterleiten, wo die Trends ermittelt werden können und wo die Prüfung gegen das Kriterium erfolgen kann. In diesem Fall ist wenigstes ein Teil der Impedanzauswertungseinheit Teil des Service-Centers. Eine Aufteilung der Impedanzauswertungseinheit auf das Patientengerät für die Vorauswertung und auf das Service-Center für die Trendauswertung und die daran anknüpfende weitere Auswertung ist ebenfalls möglich und sinnvoll. Das Service-Center kann bei festgestellter Dekompensation den behandelnden Arzt in geeigneter Weise alarmieren oder ihm die Daten zur eigenen Beurteilung in geeigneter Weise zur Verfügung stellen. Das Implantat kann auch nur die Impedanzwerte speichern und diese in geeigneten Zeitintervallen an das Patientengerät senden.

Die Bestimmung sowohl der Parameter und Trends als auch deren Prüfung gegen das Kriterium kann dann wiederum im Patientengerät oder im Service-Center erfolgen.

Eine mögliche Ausführungsvariante (Variante A) besteht in einen ICD 10 mit einer Defibrillations-Elektrode 38 im rechten Ventrikel. Hierbei erfolgt die transthorakale Impedanzmessung mittels der Stromeinspeisung über die Schockwendel 38 und das ICD-Gehäuse 42 und der Spannungsmessung über die rechtsventrikuläre Spitzen-Elektrode 18 und das ICD-Gehäuse 42 (siehe auch Figuren 1 und 2). Damit wird ein ausreichend großer Teil der Lunge vom Messpfad erfasst.

Um Energie zu sparen wird die Impedanz einmal jede Stunde für ca. 30 Sekunden mit einer hohen Abtastrate von beispielsweise 32 Messungen je Sekunde gemessen. Aus diesen Messwerten bestimmt das Implantat die mittlere Impedanz M durch Mittelung über alle Werte. Weiterhin wird über ein entsprechendes Digitalfilter das Atmungssignal aus den Messwerten herausgelöst und dessen Effektivwert bestimmt. Aus dem Effektivwert und der mittleren Impedanz wird der relative Effektivwert der Atmungsmodulation berechnet und im internen Trendspeicher abgelegt. Einmal täglich zu einer geeigneten, festen Zeit wird aus dem Trendspeicher der Tagesmittelwert des relativen Effektivwertes berechnet und in einem Langzeitspeicher abgelegt. Wenn dieser Tagesmittelwert länger als eine festgelegte Zeit stärker als ein festgelegtes Maß fällt, wird das als Indikator für eine Dekompensation bewertet. Dazu wird beispielsweise die Differenz zwischen dem aktuellen und dem vorherigen Tagesmittelwert in einer Differenzensumme aufakkumuliert, wenn der Tagesmittelwert fällt. Bleibt der Tagesmittelwert konstant oder steigt er, wird die Differenzensumme schrittweise wieder verringert. Wenn die Differenzensumme einen festgelegten Schwellwert überschreitet wird das als Indiz für eine Dekompensation gewertet und das Implantat löst einen Alarm aus. Möglich ist es auch, dass das Implantat den Trendspeicher oder den Tagesmittelwert über eine Telemetrieverbindung an ein externes Patientengerät sendet. Das Patientengerät kann dann die Berechnung und Bewertung der Parameter sowie das Auslösen eines Alarms durchführen oder die Daten an ein Service-Center weiterleiten, das dann die weitere Verarbeitung der Daten übernimmt.

Eine weiter mögliche Ausführungsvariante (Variante B) beinhaltet wiederum einen ICD 10 mit einer Defibrillations-Elektrode 38 im rechten Ventrikel. Die transthorakale Impedanzmessung erfolgt stündlich wie in Variante A. Aus den gemessenen Impedanzwerten bestimmt das Implantat die mittlere Impedanz durch Mittelung über alle Werte und über ein entsprechendes Digitalfilter das Atmungssignal und berechnet dessen Effektivwert.

Beide Werte werden in einem Trendspeicher 56 abgelegt. Der Trendspeicherinhalt wird mittels der Telemetrieeinheit 58 einmal täglich über eine Telemetrieverbindung zu einem externen Patientengerät 120 übertragen, das die Daten über eine Internetverbindung an ein Service-Center 130 weiterleitet; siehe Figur 8. Im Service-Center 130 werden die zirkadianen Zyklen für die mittlere Impedanz und den Effektivwert des Atmungssignals bestimmt. Von diesen zirkadianen Zyklen werden im Service-Center jeweils geeignete Parameter bestimmt, beispielsweise die maximalen Tagesschwankungen und die Phasenlage beider Zyklen zueinander als Zeitdifferenz zwischen den jeweiligen Maxima. Tagesschwankungen und Phasenlage werden dann mit vorgegebenen Schwellwerten, die auch patientenabhängig vorgegeben werden können, verglichen. Bei Überschreitung eines oder einer geeigneten Kombination von Schwellwerten, auch in Kombination mit anderen in der Datenbank verfügbaren Patientendaten, wird der Patientenzustand als "kritisch" eingestuft und entsprechend gekennzeichnet. Zusätzlich kann der behandelnde Arzt auf geeignete Art benachrichtigt werden. Der Arzt kann daraufhin den Gesundheitszustand des Patienten verifizieren und gegebenenfalls therapeutische Maßnahmen einleiten.

Eine weitere mögliche Ausführungsvariante (Variante C) beinhaltet ebenfalls einen ICD 10 mit transthorakaler Impedanzmessung wie in Variante A. Das Implantat 10 bestimmt wiederum stündlich die mittlere Impedanz, speichert sie in einem Trendspeicher 56 ab und überträgt sie an ein externes Patientengerät 120, das die Daten an ein Service-Center 130 weiterleitet. Im Service-Center werden dann Tagesmittelwert der mittleren Impedanz und deren zirkadianer Zyklus bestimmt. Aus dem zirkadianen Zyklus werden die Zeitdauern für die Unterschreitung (untere Halbwelle) und Überschreitung (obere Halbwelle) des Tagesmittelwerts ermittelt. Das Verhältnis der beiden Zeitdauern wird mit einem vorgegebenen Schwellwert verglichen und bei Überschreitung wird der Patientenzustand als "kritisch" eingestuft und entsprechend gekennzeichnet. Zusätzlich kann der behandelnde Arzt auf geeignete Art benachrichtigt werden. Der Arzt kann daraufhin den Gesundheitszustand des Patienten verifizieren und gegebenenfalls therapeutische Maßnahmen einleiten.

Erfindungsgemäß werden die wie zuvor beschrieben aufgenommenen Messwerte vor der weiteren Auswertung gewichtet oder verworfen.

Hierzu werden im Folgenden einige Beispiele erläutert. Es versteht sich, dass die hier beispielhaft angegebenen Auswahl- und Gewichtungs-Methoden auch in beliebigen anderen Kombinationen verwendet werden können:

Ein Verwerfen von Messwerten, deren Validität angezweifelt wird, kann wie folgt erfolgen:

Für eine Berechnung von $Zmean_h$ und $Zvar_h$ (siehe oben) werden Einzelwerte Zi verworfen, wenn deren Validität angezweifelt werden muss, weil beispielsweise eine Einzelmessung sehr stark abweicht ("Ausreißer") oder ein einzelner Herzschlag mit stark abweichender Frequenz (z. B. bei einer frühzeitigen ventrikulären Kontraktion, evtl. PVC) vorliegt. Die Messung wird entsprechend verlängert oder insgesamt für ungültig erklärt, wenn nicht genügend valide Werte für die Berechnung von $Zmean_h$ zusammenkommen.

Eine stündliche Messung kann auch ausgelassen, verschoben oder während der Aufnahme der Einzelmessungen abgebrochen werden, wenn das Ergebnis nicht die Kriterien für Validität erfüllt, weil z. B. zu starke Variabilität $Zvar_h$ vorliegt oder ein zu hoher Wert von Accelerometersignal, Atmungssignal, CLS-Rate oder Herzfrequenz. Auch kann eine stündliche Messung ausgelassen, verschoben oder während der Aufnahme der Einzelmessungen abgebrochen werden, wenn eine ventrikuläre Tachykardie vorliegt, eine antitachykarde Therapie statt findet oder eine zu hohe Varianz der Einzelmessungen vorliegt.

Eine Vergabe von hoher Gewichtung für Messungen mit kleiner Varianz kann beispielsweise wie folgt erfolgen:

Gemäß einem ersten Beispiel erfolgt die Gewichtung der Stundenmittelwerte mit der "Stabilität" der Messungen, z. B. mit dem Kehrwert der Varianz. Dies ergibt einen Parameter $Zmean_{d\_w}$, der Messungen unter stabilen Bedingungen stärker berücksichtigt:

z. B.:

$$Zmean_{d\_w} = \sum_i \frac{Zmean_h(i)}{Zvar_h(i)} \Big/ \sum_i \frac{1}{Zvar_h(i)}$$

Eine Auswahl oder eine hohe Gewichtung von Messungen $Zmean_h$ kann auch erfolgen, wenn die Impedanzwerte unter Bedingungen aufgenommen werden, die besonders empfindlich gegenüber dem zu beobachtenden Effekt sind. Dabei werden Messungen mit hoher Vergleichbarkeit der Bedingungen bevorzugt. Dies wird nachfolgend anhand mehrerer Beispiele näher erläutert:

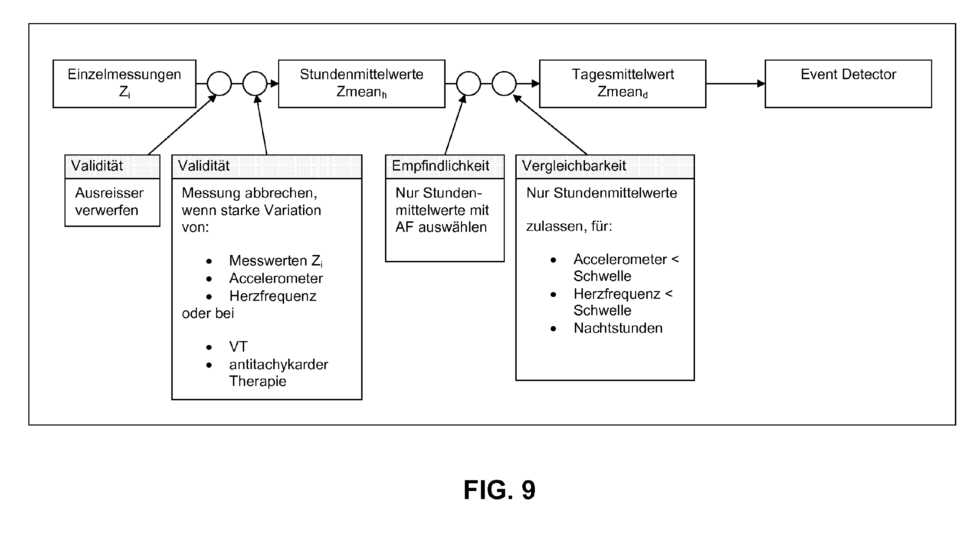

Ein zweites Beispiel, welches in Figur 9 bildlich dargestellt ist, betrifft die Auswahl von Messungen während atrialer Fibrillation AF:

Messungen während AF werden ausgewählt und gesondert gemittelt zu einem Tagesmittelwert unter AF: $Zmean_{d\_AF}$

Nur die $Zmean_h$, die folgende zusätzlichen Bedingungen erfüllen, werden dabei berücksichtigt (für die Vergleichbarkeit):

i. Accelerometer-Signal unter einer Schwelle (Patient in Ruhe)
ii. Herzfrequenz unter einer Schwelle (Patient in Ruhe)
iii. Nachtzeit

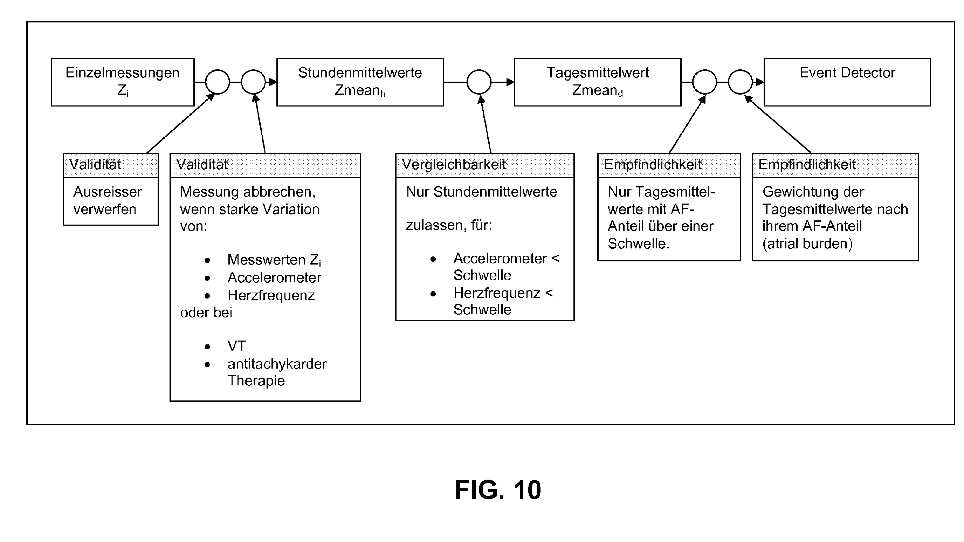

Ein drittes Beispiel, welches in Figur 10 bildlich dargestellt ist, illustriert eine hohe Gewichtung von Tagesmittelwerten mit hohem AF-Anteil. Hierzu erfolgt zunächst eine Bestimmung von Tagesmittelwerten $Zmean_d$ aus $Zmean_h$, die folgende Bedingungen erfüllen (für die Vergleichbarkeit):

i. das Accelerometer-Signal liegt unterhalb einer Schwelle (d. h. der Patient befindet sich in Ruhe)
ii. die Herzfrequenz liegt unterhalb einer Schwelle (d. h. der Patient befindet sich in Ruhe)

Anschließend erfolgt eine Gewichtung der Tagesmittelwerte $Zmean_d$ nach ihrem Anteil von AF während dieses Tages (atrial burden A) für die weitere Verarbeitung. Dies kann beispielsweise nach folgender Vorschrift geschehen:

$$P = \sum_i A_i * Zmean_{d,i} \bigg/ \sum_i A_i$$

Ein viertes Beispiel erläutert die Auswahl von Messungen bei hoher körperlicher Belastung. Hierfür werden Messungen mit Accelerometersignal über einer bestimmten Schwelle ausgewählt und, mit ihrem Accelerometersignal-Level gewichtet, zu einem Tagesmittelwert unter Belastung: $Zmean_{d\_Load}$ gemittelt. Hierbei werden nur $Zmean_h$, die folgende zusätzlichen Bedingungen erfüllen, berücksichtigt (für die Vergleichbarkeit):

i. Herzfrequenz in einem vorbestimmten (erhöhten) Bereich
ii. Messung während der Tagesstunden (z. B. 08:00 - 20:00 Uhr).

Ein fünftes Beispiel betrifft die Auswahl von Messungen in der Nacht. Hierfür werden Messungen zur Nachtzeit (z. B. 23:00 - 04:00 Uhr) ausgewählt und zu einem täglichen Mittelwert $Zmean_{d\_Night}$ gemittelt. Dabei werden nur $Zmean_h$ berücksichtigt, die folgende zusätzlichen Bedingungen erfüllen (für die Vergleichbarkeit):

i. Herzfrequenz in einem vorbestimmten, niedrigen Bereich (z. B. 40-80 bpm)
ii. Accelerometersignal unter einer Schwelle
iii. keine AF
iv. geringe Varianz der Atmungsparameter (regelmäßige Atmung)

Ein sechstes Beispiel betrifft die Auswahl von Messungen zu einer bestimmten Tageszeit. Hierfür werden Messungen in einem Tageszeitbereich (z. B. 14:00 - 17:00 Uhr) ausgewählt und zu einem täglichen Mittelwert $Zmean_{d\_Daytime}$ gemittelt. Dabei werden nur $Zmean_h$ berücksichtigt, die folgende zusätzlichen Bedingungen erfüllen (für die Vergleichbarkeit):

i. Herzfrequenz in einem vorbestimmten, niedrigen Bereich (z. B. 60-90 bpm), d. h. Patient in Ruhe.
ii. Accelerometersignal unter einer Schwelle
iii. keine AF

Es können natürlich auch mehrere solcher Auswertungen parallel durchgeführt und als separate "Kanäle" dem Ereignis-Detektor zugeführt werden.

Die Bewertungs- und Verarbeitungseinheit im Sinne der Erfindung kann durch die Steuereinheit 54 verkörpert sein. Alternativ kann die Bewertungs- und Verarbeitungseinheit auch Bestandteil des Service-Centers 130 sein. Die Bewertungs- und Verarbeitungseinheit kann (zumindest in Teilen) auch Bestandteil der Impedanz-Auswerteeinheit 78 sein.

Eingangswerte der Bewertungs- und Verarbeitungseinheit sind die Ausgangswerte des Aktivitätssensors 60 und des Tageszeit-Gebers 80. Weiterhin können das ClosedLoop-Frequenzanpassungssignal, Atmungsparameter (aus Impedanzmessungen 76 70), Arrhythmieparameter (aus CTRL 54), oder der Positur-Sensor 82 Eingangswerte liefern. Auf diese Weise kann die Wichtung der Messwerte in Abhängigkeit der Stärke einer körperlichen Aktivität und/oder der Tageszeit, etc. erfolgen. Darüber hinaus ist die Bewertungs- und Verarbeitungseinheit ausgebildet, die Messwerte selbst auszuwerten, um sie in Abhängigkeit des Auswertungsergebnisses zu gewichten.

Die Bewertungs- und Verarbeitungseinheit ist ausgebildet, über die Impedanzerfassungs- und Auswerteeinheit erfasste Messwerte von Impedanzsignalen und/oder über die einzelne Sensingeinheit aufgenommene Messwerte von intrakardialen Elektrokardiogrammsignalen nach Auswertung des jeweiligen Signalverlaufs und ggf. unter Berücksichtung der Ausgangssignale des Positursensors und des Tageszeitgebers zu gewichten und nach Gewichtung zum Erfassen kardialer Dekompensationen weiterzuverarbeiten.

**Patentansprüche**

1. Monitoring-Vorrichtung mit einem Signaleingang für Signale, die Messwerte eines oder mehrerer physiologischer Parameter repräsentieren und einer mit dem Signaleingang verbundenen Bewertungs- und Verarbeitungseinheit, **dadurch gekennzeichnet, dass** die Bewertungs- und Verarbeitungseinheit ausgebildet ist, unter eingehenden Messwerten einzelne Werte für die weitere Verarbeitung nach einem oder mehreren Kriterien derart auszuwählen oder unterschiedlich zu gewichten, dass Messwerte ausgewählt oder nicht ausgewählt werden und ausgewählte Messwerte mit hoher Gewichtung oder mit niedriger Gewichtung versehen werden, je nachdem ob vorher eingestellte oder adaptive Grenzwerte für Messwerte und/oder vorher eingestellte oder adaptive Grenzwerte für Änderungen oder Abweichungen gegenüber bzw. von zuvor aufgenommenen Messwerten eingehalten, überschritten oder unterschritten werden, und eine Auswertung der Messwerte abgebrochen wird, wenn ein jeweiliger Messwert auf Grund der Gewichtung unterhalb einer vorgegebenen oder adaptiven Schwelle liegen wird.

2. Monitoring-Vorrichtung nach Anspruch 1, **dadurch gekennzeichnet, dass** die Bewertungs- und Verarbeitungseinheit ausgebildet ist, in dem Fall, dass vor der Durchführung einer Messung bereits aus Messwerten anderer Parameter oder aus den bisher erfolgten Teilen der Messung eine Gewichtung unterhalb einer bestimmten Schwelle zu erwarten ist, eine Messung abgebrochen wird oder ganz entfällt.

3. Monitoring-Vorrichtung nach Anspruch 1 oder 2, **dadurch gekennzeichnet, dass** die Bewertungs- und Verarbeitungseinheit ausgebildet ist, im Fall einer abgebrochenen Auswertung oder einer entfallenen Messung, innerhalb eines vorgegebenen Wiederholungszeitraums nach der ersten Messung, diese Messung zu wiederholen, sobald die Kriterien für das Entfallen der Messung nicht mehr erfüllt sind.

4. Monitoring-Vorrichtung nach einem der Ansprüche 1 bis 3, **dadurch gekennzeichnet, dass** die Bewertungs- und Verarbeitungseinheit ausgebildet ist, unter den eingehenden Werten einzelne Werte für die weitere Verarbeitung nach einem oder mehreren der folgenden Kriterien derart auszuwählen oder unterschiedlich zu gewichten, dass Messwerte, deren Validität angezweifelt werden muss, nicht ausgewählt oder mit sehr niedriger Gewichtung versehen werden, wenn:

   - die Variabilität eines Messwerts der selbst ein Mittelwert aus vielen Einzelwerten ist, über einem vorgegebenen entsprechendem Grenzwert liegt,
   - ein die Aktivität eines Patienten kennzeichnendes Sensorsignal wie ClosedLoopStimulations-Ratensteuersignal, Akzelerometerausgangswert, Atemfrequenz, Atemtiefe, und/oder Minutenvolumen über einem vorgegebenen entsprechendem Grenzwert liegt,
   - ein Signal oder mehrere Signale einen instabilen Zustand eines Patienten wie eine ventrikuläre Tachykardie anzeigt, oder
   - eine antiachykarde Therapie ausgeführt wird.

5. Monitoring-Vorrichtung nach einem der Ansprüche 1 bis 4, **dadurch gekennzeichnet, dass** die Bewertungs- und Verarbeitungseinheit ausgebildet ist, unter den eingehenden Werten einzelne Werte für die weitere Verarbeitung nach einem oder mehreren Kriterien derart auszuwählen oder unterschiedlich zu gewichten, dass Messwerte unter Bedingungen, die eine gute Vergleichbarkeit ermöglichen, mit einer hohen Wichtung versehen und bevorzugt ausgewertet werden.

6. Monitoring-Vorrichtung nach einem der Ansprüche 1 bis 5, **dadurch gekennzeichnet, dass** die Bewertungs- und Verarbeitungseinheit ausgebildet ist, Messwerte, die in einem vorgegebenen Tageszeitbereich aufgenommen werden, mit einer hohen Wichtung zu versehen und bevorzugt auszuwerten.

7. Monitoring-Vorrichtung nach Anspruch 5, **dadurch gekennzeichnet, dass** die Bewertungs- und Verarbeitungseinheit ausgebildet ist, Messwerte, die einem vorgegebenen Herzfrequenzbereich zugeordnet sind, mit einer hohen Wichtung zu versehen und bevorzugt auszuwerten und/oder Messwerte, die einem vorgegebenen Belastungsbereich (psychisch oder physisch) zugeordnet sind, mit einer hohen Wichtung zu versehen und bevorzugt auszuwerten und/oder Messwerte, die mit einem Anteil atrialen Flimmerns innerhalb einer bestimmten Zeitspanne einhergehen, der unterhalb oder oberhalb eines vorgegebenen Grenzwertes liegt, mit einer hohen Wichtung zu versehen und bevorzugt auszuwerten und/oder Messwerte, die mit einer Atmungsamplitude und Atemfrequenz in einem vorbestimmten oder adaptiv ermittelten Intervall einhergehen, mit einer hohen Wichtung zu versehen und bevorzugt auszuwerten.

8. Monitoring-Vorrichtung nach einem der Ansprüche 1 bis 7, **dadurch gekennzeichnet, dass** die Bewertungs- und Verarbeitungseinheit ausgebildet ist, unter den eingehenden Werten einzelne Werte für die weitere Verarbeitung nach einem oder mehreren Kriterien derart auszuwählen oder unterschiedlich zu gewichten, dass Messwerte die unter Bedingungen gewonnen wurden, bei denen das Messsignal eine hohe Empfindlichkeit gegenüber dem zu beobachtenden Effekt zeigt, bevorzugt ausgewertet und mit einer hohen Wichtung versehen werden.

9. Monitoring-Vorrichtung nach Anspruch 8, **dadurch gekennzeichnet, dass** die Bewertungs- und Verarbeitungseinheit ausgebildet ist, Messwerte, die mit einem Anteil atrialen Flimmerns innerhalb einer bestimmten Zeitspanne einhergehen, der oberhalb eines vorgegebenen Grenzwertes liegt, mit einer hohen Wichtung zu versehen und bevorzugt auszuwerten und/oder Messwerte, die unter hoher körperlicher, physischer, oder psychischer Belastung aufgenommen werden, mit einer hohen Wichtung zu versehen und bevorzugt auszuwerten und/oder Messwerte, die in einem vorgegebenen Herzfrequenz-Bereich aufgenommen werden, mit einer hohen Wichtung zu versehen und bevorzugt auszuwerten.

10. Monitoring-Vorrichtung nach Anspruch 8, mit einem Eingang für ein Positursensorsignal, das eine liegende oder aufrechte Positur anzeigt, **dadurch gekennzeichnet, dass** die Bewertungs- und Verarbeitungseinheit ausgebildet ist, Messwerte, die in einer vorher festgelegten Positur aufgenommen werden, mit einer hohen Wichtung zu versehen und bevorzugt auszuwerten.

11. Monitoring-Vorrichtung nach Anspruch 8, mit einem Eingang für ein Positursensorsignal, das eine liegende oder aufrechte Positur anzeigt, und einem Eingang für ein Tageszeitsignal, **dadurch gekennzeichnet, dass** die Bewertungs- und Verarbeitungseinheit ausgebildet ist, Messwerte, die morgens nach Wechsel von einer liegenden Positur zu einer aufrechten Positur oder abends nach Wechsel von einer aufrechten Positur zu einer liegenden Positur aufgenommen werden, mit einer hohen Wichtung zu versehen und bevorzugt auszuwerten.

12. Monitoring-Vorrichtung nach einem der Ansprüche 1 bis 11, **dadurch gekennzeichnet, dass** die Bewertungs- und Verarbeitungseinheit mit einer Impedanzerfassungseinheit zum Bestimmen eines Impedanzsignals, das einen intrakardialen Impedanzverlauf repräsentiert und einer Impedanzauswertungseinheit verbunden ist, die aus dem zeitlichen Verlauf des Impedanzsignals ein Aktivitätssignal bestimmt, welches hohe körperliche oder physische Belastung anzeigt.

13. Monitoring-Vorrichtung nach einem der Ansprüche 1 bis 12, **dadurch gekennzeichnet, dass** die Bewertungs- und Verarbeitungseinheit zur Verarbeitung von Messwerten eines oder mehrerer der folgenden Parameter ausgebildet ist: Biomarker, Elektrolyte, Glukose, Hämatokrit, Blutwiderstand, Temperatur und/oder Aussendruck.

14. Monitoring-Vorrichtung nach einem der Ansprüche 1 bis 13, **dadurch gekennzeichnet, dass** die Signalerfassungseinheit und die Bewertungs- und Verarbeitungseinheit im Implantat integriert sind oder die Signaleinheit im Implantat integriert ist und die Bewertungs- und Verarbeitungseinheit in einem mit dem Implantat kommunizierenden externen Gerät integriert ist oder die Signaleinheit im Implantat integriert ist und die Bewertungs- und Verarbeitungseinheit in einem mit dem Implantat indirekt kommunizierenden Service Center integriert ist.

15. Verfahren zum Verarbeiten von Messwerten physiologischer Parameter, **dadurch gekennzeichnet, dass** unter den eingehenden Werten einzelne Werte für die weitere Verarbeitung nach einem oder mehreren der folgenden Kriterien derart ausgewählt oder unterschiedlich gewichtet werden, dass Messwerte, deren Validität angezweifelt werden muss, nicht ausgewählt oder mit sehr niedriger Gewichtung versehen werden, wenn:

- die Variabilität eines Messwerts, der selbst ein Mittelwert aus vielen Einzelwerten ist, über einem vorgegebenen entsprechendem Grenzwert liegt,
- ein die Aktivität eines Patienten kennzeichnendes Sensorsignal wie ClosedLoopStimulations-Ratensteuersignal, Akzelerometerausgangswert, Atemfrequenz, Atemtiefe, und/oder Minutenvolumen über einem vorgegebenen entsprechenden Grenzwert liegt,
- ein Signal oder mehrere Signale einen instabilen Zustand eines Patienten wie eine ventrikuläre Tachykardie anzeigt, oder
- eine antitachykarde Therapie ausgeführt wird.

**FIG. 1**

EP 2 241 249 A1

FIG. 2

FIG. 3

**FIG. 4**

RV-RING
LV-RING
RV-TIP
LV-TIP

SW1, SW2, SW3, SW4

72, 74, 76, 78, 80, 82

IMP
IMP-EVAL
POS
MEM
TEL
CTRL
ACT
RV-STIM / RV-SENS
LV-STIM / LV-SENS

50, 52, 54, 56, 58, 60, 70

FIG. 5

FIG. 6

Tageszeit h

Z

**FIG. 7**

FIG. 8

Einzelmessungen $Z_i$

Stundenmittelwerte $Zmean_h$

Tagesmittelwert $Zmean_d$

Event Detector

Validität

Ausreisser verwerfen

Validität

Messung abbrechen, wenn starke Variation von:

- Messwerten $Z_i$
- Accelerometer
- Herzfrequenz

oder bei

- VT
- antitachykarder Therapie

Empfindlichkeit

Nur Stunden-mittelwerte mit AF auswählen

Vergleichbarkeit

Nur Stundenmittelwerte

zulassen, für:

- Accelerometer < Schwelle
- Herzfrequenz < Schwelle
- Nachtstunden

**FIG. 9**

| Einzelmessungen $Z_i$ | ○—○ | Stundenmittelwerte $Zmean_h$ | ○ | Tagesmittelwert $Zmean_d$ | ○—○ | Event Detector |

**Validität**

Ausreisser verwerfen

**Validität**

Messung abbrechen, wenn starke Variation von:

- Messwerten $Z_i$
- Accelerometer
- Herzfrequenz

oder bei

- VT
- antitachykarder Therapie

**Vergleichbarkeit**

Nur Stundenmittelwerte

zulassen, für:

- Accelerometer < Schwelle
- Herzfrequenz < Schwelle

**Empfindlichkeit**

Nur Tagesmittel-werte mit AF-Anteil über einer Schwelle.

**Empfindlichkeit**

Gewichtung der Tagesmittelwerte nach ihrem AF-Anteil (atrial burden)

## FIG. 10

Europäisches
Patentamt

European
Patent Office

Office européen
des brevets

**EUROPÄISCHER RECHERCHENBERICHT**

Nummer der Anmeldung

EP 10 15 6626

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2006/293609 A1 (STAHMANN JEFFREY E [US] ET AL) 28. Dezember 2006 (2006-12-28) * Absätze [0056] - [0058], [0096]; Abbildung 7 * ----- | 1-15 | INV. A61B5/00 A61B5/021 |
| X | US 2003/074029 A1 (DENO D CURTIS [US] ET AL DENO D CURTIS [US] ET AL) 17. April 2003 (2003-04-17) * Absätze [0067], [0074], [0077], [0088], [0089], [0175], [0178] * ----- | 1,15 | |
| X | US 2005/240087 A1 (KEENAN DESMOND B [US] ET AL) 27. Oktober 2005 (2005-10-27) * das ganze Dokument * ----- | 1,15 | |
| A | US 5 092 343 A (SPITZER ROBERT [US] ET AL) 3. März 1992 (1992-03-03) * das ganze Dokument * ----- | 1-15 | |
| A | US 2005/043644 A1 (STAHMANN JEFFREY E [US] ET AL) 24. Februar 2005 (2005-02-24) * das ganze Dokument * ----- | 1-15 | RECHERCHIERTE SACHGEBIETE (IPC) A61B |
| A | US 2008/242955 A1 (UUTELA KIMMO [FI] ET AL) 2. Oktober 2008 (2008-10-02) * das ganze Dokument * ----- | 1-15 | |
| A | US 2003/117296 A1 (SEELY ANDREW J E [CA]) 26. Juni 2003 (2003-06-26) * das ganze Dokument * ----- | 1-15 | |

Der vorliegende Recherchenbericht wurde für alle Patentansprüche erstellt

| Recherchenort | Abschlußdatum der Recherche | Prüfer |
|---|---|---|
| München | 2. Juni 2010 | Schöffmann |

**ANHANG ZUM EUROPÄISCHEN RECHERCHENBERICHT
ÜBER DIE EUROPÄISCHE PATENTANMELDUNG NR.**

EP 10 15 6626

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-06-2010

| Im Recherchenbericht angeführtes Patentdokument | Datum der Veröffentlichung | Mitglied(er) der Patentfamilie | Datum der Veröffentlichung |
|---|---|---|---|
| US 2006293609 A1 | 28-12-2006 | KEINE | |
| US 2003074029 A1 | 17-04-2003 | CA 2433359 A1 | 11-07-2002 |
| | | DE 60132016 T2 | 11-12-2008 |
| | | EP 1347704 A2 | 01-10-2003 |
| | | JP 4160391 B2 | 01-10-2008 |
| | | JP 2004523269 T | 05-08-2004 |
| | | WO 02053026 A2 | 11-07-2002 |
| US 2005240087 A1 | 27-10-2005 | AU 2004290588 A1 | 02-06-2005 |
| | | CA 2545881 A1 | 02-06-2005 |
| | | EP 1684626 A2 | 02-08-2006 |
| | | JP 2007521852 T | 09-08-2007 |
| | | WO 2005048824 A2 | 02-06-2005 |
| US 5092343 A | 03-03-1992 | KEINE | |
| US 2005043644 A1 | 24-02-2005 | US 2008221468 A1 | 11-09-2008 |
| US 2008242955 A1 | 02-10-2008 | DE 102008016298 A1 | 02-10-2008 |
| | | NL 2001399 C2 | 22-09-2009 |
| | | NL 2001399 A1 | 02-10-2008 |
| US 2003117296 A1 | 26-06-2003 | AU 7224901 A | 14-01-2002 |
| | | WO 0202006 A1 | 10-01-2002 |
| | | CA 2418003 A1 | 10-01-2002 |
| | | US RE41236 E1 | 20-04-2010 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5957861 A
- US 6076015 A
- US 6454719 B
- US 20060041280 A
- US 20060258952 A
- US 20060264776 A
- EP 1665983 A
- US 5876353 A
- US 6449509 B
- EP 1510173 A

**In der Beschreibung aufgeführte Nicht-Patentliteratur**

- **Yu CM ; Wang L ; Chau E ; Chan RH ; Kong SL ; Tang MO ; Christensen J ; Stadler RW ; Lau CP.** Intrathoracic impedance monitoring in patients with heart failure: correlation with fluid status and feasibility of early warning preceding hospitalization. *Circulation,* 2005, vol. 112 (6), 841-8
- Braunwald's Heart Disease. Elsevier, 2005
- **Zima, E. et al.** Determination of left ventricular volume changes by intracardiac conductance using a biventricular electrode configuration. *Europace,* 2006, vol. 8.7, 537-44
- **Stahl, C. et al.** Intracardiac Impedance Monitors Hemodynamic Deterioration in a Chronic Heart Failure Pig Model. *J.Cardiovasc.Electrophysiol.,* 2007, vol. 18, 985-90